Europäisches Patentamt

(19) European Patent Office

Office européen des brevets

(11) Publication number: **0 001 660**
**B1**

(12) **EUROPEAN PATENT SPECIFICATION**

(21) Application number: **78200237.2**

(22) Date of filing: **11.10.78**

(51) Int. Cl.³: **C 07 C 45/36, C 07 C 47/54**

(54) Process for the purification of benzaldehyde.

(30) Priority: **18.10.77 NL 7711393**

(43) Date of publication of application:
**02.05.79 Bulletin 79/9**

(45) Publication of the grant of the European patent:
**25.03.81 Bulletin 81/12**

(84) Designated Contracting States:
**BE DE FR GB**

(56) References cited:
**DE - B - 1 236 493**
**US - A - 3 931 330**

**Ullmann "Encyklopädie der technischen Chemie"**
**4th. edition, 1974.**
**Verlag CHEMIE, Weinheim-Bergstraße, vol. 8,**
**page 345.**

**Chemical Abstracts vol. 82, no. 11, 1975.**
**Columbus, Ohio, USA**
**K. OKUNO et al "Benzaldehyde of high purity",**
**page 408, abstract no. 72 638e.**

**Chemical Abstracts vol. 85, no. 19, 1976.**
**Columbus, Ohio, USA.**
**J. GASPERIK "Benzaldehyde oxidation test, a**
**model reaction with radical mechanism, II. The**
**purification of benzaldehyde and stabilization of**
**its activity", page 495, abstract no. 142 755 d.**

(73) Proprietor: **STAMICARBON B.V.**
**Postbus 10**
**NL-6160 MC Geleen (NL)**

(72) Inventor: **Elmendorp, Jan**
**Lindestraat 152**
**NL-6444 AS Brunssum (NL)**

(74) Representative: **Hoogstraten, Willem Cornelis**
**Roeland et al,**
**OCTROOIBUREAU DSM Postbus 9**
**NL-6160 MA Geleen (NL)**

Process for the purification of benzaldehyde

The invention relates to a process for the purification of benzaldehyde prepared by oxidation of toluene with a gas containing molecular oxygen.

Benzaldehyde is an important starting material in chemical synthesis, e.g. the synthesis of scents and flavours. For these applications the benzaldehyde is often required to have a high degree of purity. However, benzaldehyde prepared by oxidation of toluene with a gas containing molecular oxygen, contains annoying impurities that are very difficult to remove. It is particularly difficult to obtain a product from crude benzaldehyde that meets the demands of olfactory specifications. The presence of impurities manifests itself in the quite rapid discolouration of the benzaldehyde upon storage, which occurs already at a very low concentration (p.p.m. by weight level) of the impurities.

It has been proposed (Japanese Patent Publication 24.467/74) to purify crude benzaldehyde by treating it with an aqueous solution of sodium hydroxide. This method of purification, however, does not give satisfactory results. The benzaldehyde treated is still found to discolour quite soon.

The invention aims at providing a solution to said problem. According to the invention, pure benzaldehyde is prepared by treating impure benzaldehyde with an oxidizing agent and disʼilling it.

It is particularly surprising that crude benzaldehyde can be purified in this way. For, it is common knowledge that oxidizing agents can readily oxidize benzaldehyde and it is coloured reaction products that may form then (see, e.g., Beilsteins Hardbuch der Organischen Chemie, 4. Auflage, 7, 179—180 and 7—111, 811). In the treatment according to the invention the loss of benzaldehyde is very small and may be of the order of 0.5 to 1% by weight. Benzaldehyde meeting the demands of olfactory specifications can be obtained, even from crude benzaldehyde prepared by oxidation of toluene.

Suitable oxidizing agents in the process according to the invention are, e.g., gases containing molecular oxygen, e.g. air, pure oxygen and nitrogen-oxygen mixtures with a different composition from air, and especially peroxide agents. Other oxidizing agents, such as, e.g., potassium permanganate or potassium (di)chromate, are slightly less suitable. The oxidizing agents are used by preference in amounts between 1 and 5000 mmoles, calculated as active oxygen, per kg benzaldehyde.

Suitable amounts of the gas containing molecular oxygen range between 0.1 and 5, preferably between 0.5 and 2, litres (N.T.P.), calculated as molecular oxygen, per hour and per kg of benzaldehyde. Larger amounts may be used, but offer no advantage. The treating time may range, for instance, between 0.5 and 10 hours.

The peroxide agent used by preference is hydrogen peroxide, e.g. as a 30% by weight aqueous solution, which is a normal commercial product. If so desired, a more dilute aqueous solution may also be used, e.g. a 2—30% by weight solution. Also more concentrated aqueous solutions, of up to even 90% by weight or more, may be used, but feed control will be more difficult. The amount of hydrogen peroxide (calculated as $H_2O_2$ relative to the benzaldehyde preferably ranges between 3 and 1500 mmoles per kg (about 0.01—5% by weight of hydrogen peroxide). Larger amounts of hydrogen peroxide may be used, but they will only raise the treating cost. Use is preferably made of an amount of hydrogen peroxide of 3 to 150 mmoles per kg of benzaldehyde.

Examples of other suitable peroxide agents are percarboxylic acids and their salts, e.g. perbenzoic acid or peracetic acid, and inorganic persalts, e.g. potassium persulphate. The amounts of these to be used, calculated as moles of peroxide per kg of benzaldehyde, correspond to those of hydrogen peroxide.

The treatment of the crude benzaldehyde with an oxidizing agent may be effected in the liquid phase at temperatures of, e.g., 20 to 200°C. If a peroxide agent is used as the oxidizing agent, approximately room temperature or elevated temperature can well be used; if a gas containing molecular oxygen is used as the oxidizing agent, the use of elevated temperature, preferably between 75 and 175°C, is desirable. Higher temperatures than 200°C may be used, but will give rise to undesirable great losses of benzaldehyde, and they are less desirable for reasons of safety. The reaction pressure is less critical and may range, for instance, between 10 and 1000 kPa. A pressure approximately equal to atmospheric pressure, e.g. of between 50 and 200 kPa, is to be preferred for practical considerations.

The benzaldehyde treated with an oxidizing agent is then distilled. If so desired, the treatment with an oxidizing agent and the distillation may be effected simultaneously. The distillation may be effected at atmospheric or elevated pressure, but, preferably, at reduced pressure, e.g. a pressure of 2—35 kPa. If hydrogen peroxide is used as the oxidizing agent, an important advantage of the process according to the invention lies in the fact that the only by-product with a low boiling point, water, which, in practice, is usually also contained in the hydrogen peroxide as a diluting agent for that matter, can be separated during the distillation in a simple way as the water-benzaldehyde azeotrope with a lower boiling point than benzaldehyde.

It may be advantageous to decompose any unconverted peroxide agent before the distillation. This may be done thermally, e.g. by heating the benzaldehyde containing the peroxide agent to a temperature of, e.g. 100—200°C, more in particular 120—160°C. If so desired, a catalyst for the decomposition of peroxides may be added in this step, e.g. a heavy-metal salt, such as a cobalt salt or an iron salt. Amounts of heavy metal of 0.1—20, more in particular 0.5—2 p.p.m. by weight relative to the benzaldehyde are sufficient for a satisfactory catalytic action. Larger amounts may be used, if so desired.

The invention will be elucidated with reference to the following non-restricting examples and comparative experiments. The colour values in degrees Hazen (°H) were determined by ASTM D1209/62.

### Example I

A sample of crude benzaldehyde prepared by oxidation of toluene in the liquid phase by means of a gas containing molecular oxygen with the use of a homogeneous cobalt catalyst was distilled in a sieve-tray column with 30 trays at a top pressure of 20 kPa and with a reflux ratio of 1:3.

During the distillation 5 litres (N.T.P.) of air per litre of liquid benzaldehyde were passed through per hour. The colour value of the main fraction was lower than the detection limit of 5°H. After 20 days storage in a dark-brown bottle under a nitrogen atmosphere, the colour value was still found to be no more than 5°H. The colour value of the crude benzaldehyde used as the starting product was much higher than the limit of 30°H.

### Example II

A sample of the same liquid crude benzaldehyde as used in Example I was treated for 1 hour at 140°C with a 0.5%-by-weight hydrogen peroxide in the form of a 30%-by-weight aqueous solution. Next, 5 p.p.m. by weight of cobalt (as acetate) were added and the mixture was distilled under the same conditions as in example I, but without air being passed through. The colour value of the main fraction was below the 5°H limit and was 5°H upon 20 days' storage in the way described in Example I.

### Example III

A sample of the same liquid crude benzaldehyde as used in Example I was heated at 160°C for 2 hours while 10 litres (N.T.P.) of air per litre of benzaldehyde were passed through per hour. The mixture was then distilled in a sieve-tray column with 30 trays at a top pressure of 13 pKa and with a reflux ratio of 1:3 without air being passed through. The colour value of the main fraction was below the 5°H limit. The colour value was still below this limit upon 14 days' storage in the way described in Example I.

### Example IV

A sample of the same liquid crude benzaldehyde as used in Example I was treated at 140°C for 1 hour with a 2%-by-weight perbenzoic acid. Next, 5 p.p.m. by weight of cobalt (as acetate) were added and the mixture was distilled under the same conditions as in Example II. The colour value of the main fraction was below the 5°H limit, even upon 14 days' storage in the way described in Example I.

### Comparative experiment A

A sample of the same liquid crude benzaldehyde as used in Example I was distilled without the use of an oxidizing agent, but under otherwise similar conditions. The colour value of the main fraction was 25°H and had risen to far over the limit of the determination (30°H) after 10 days' storage in the way described in Example I.

### Comparative experiment B

A sample of the same crude benzaldehyde as used in Example I was stirred at 25°C for 30 minutes with half the weight of 2.5%-by-weight aqueous sodium-hydroxide solution. The benzaldehyde layer was separated and distilled. The colour value of the main fraction was far over 30°H.

## Claims

1. A process for the preparation of pure benzaldehyde, characterized in that impure benzaldehyde which has been prepared by oxidation of toluene with a gas containing molecular oxygen is treated with an oxidizing agent and distilled.

2. Process according to claim 1, characterized in that the treatment with an oxizing agent and the distillation are effected simultaneously.

3. Process according to claim 1 or 2, characterized in that the oxidizing agent is a peroxide agent.

4. Process according to claim 3, characterized in that the oxidizing agent is hydrogen peroxide.

5. Process according to claim 3 or 4, characterized in that the amount of peroxide agent used is 3—1500 mmoles of peroxide per kg of benzaldehyde.

6. Process according to any one of the claims 3—5, characterized in that unconverted peroxide agent, if any, is decomposed before the distillation.

7. Process according to claim 1 or 2, characterized in that the oxidizing agent is a gas containing molecular oxygen.

8. Process according to claim 7, characterized in that the oxidizing agent is air.

9. Process according to claim 7 or 8, characterized in that 0.1 to 5 litres (N.T.P.), calculated as molecular oxygen, per kg of benzaldehyde and per hour are passed through for a period of 0.5 to 10 hours.

10. Process according to any one of the claims 1—9, characterized in that the treatment with an oxidizing agent is effected at a temperature of 20 to 200°C.

**Patentansprüche**

1. Verfahren zu der Herstellung von reinem Benzaldehyd, dadurch gekennzeichnet, dass verunreinigtes Benzaldehyd, gewonnen durch Oxidation von Toluol mit einems Molekularsaeurstoff enthaltenden Gas mit einem Oxidationsmittel behandelt und anschliessend destilliert wird.

2. Verfahren gemäss Anspruch 1, dadurch gekennzeichnet, dass man die Behandlung mit Oxidationsmittel und die Destillation gleichzeitig durchführt.

3. Verfahren gemäss Anspruch 1 oder 2, dadurch gekennzeichnet, dass es sich beim Oxidationsmittel un ein Peroxidagens handelt.

4. Verfahren gemäss Anspruch 3, dadurch gekennzeichnet, dass das Oxidationsmittel Wasserstoffperoxid ist.

5. Verfahren gemäss Anspruch 3 oder 4, dadurch gekennzeichnet, dass Peroxidagens in einer Menge von 3—1500 mMol Peroxid je kg' Benzaldehyd verwendet wird.

6. Verfahren gemäss einem der Ansprüche 3—5, dadurch gekennzeichnet, dass das ggf. nicht umgesetzte Peroxidagens vor Destillation abgebaut wird.

7. Verfahren gemäss Anspruch 1 oder 2, dadurch gekennzeichnet, dass das Oxidationsmittel ein Molekularsauerstoff enthaltendes Gas ist.

8. Verfahren gemäss Anspruch 7, dadurch gekennzeichnet, dass es sich beim Oxidationsmittel um Luft handelt.

9. Verfahren gemäss Anspruch 7 oder 8, dadurch gekennzeichnet, dass in einem Zeitraum von 0,5 bis 10 Stunden 0,1 bis 5 N1, berechnet als Molekularsauerstoff, je Stunde unk je kg Benzaldehyd durchgeleitet werden.

10. Verfahren gemäss einem der Ansprüche 1—9, dadurch gekennzeichnet, dass man die Behandlung mit einem Oxidationsmittel bei einer Temperatur von 20 bis 200°C vornimmt.

**Revendications**

1. Procédé de préparation de benzaldéhyde pur, caractérisé en ce que du benzaldéhyde souillé, préparé par l'oxydation de toluène avec un gaz contenant de l'oxygène moléculaire, est traité avec un agent d'oxydation et distillé ensuite.

2. Procédé selon la revendication 1, caractérisé en ce que le traitement avec l'agent d'oxydation et la distillation sont effectués en même temps.

3. Procédé selon l'une des revendications 1 à 2, caractérisé en ce que l'agent d'oxydation est un agent de peroxyde.

4. Procédé selon la revendication 3, caractérisé en ce que l'agent d'oxydation est du peroxyde d'hydrogène.

5. Procédé selon l'une des revendications 3 à 4, caractéisé en ce qu'on utilise une quantité d'agent de peroxyde de 3—1500 mmoles de peroxyde par kg de benzaldéhyde.

6. Procédé selon l'une des revendications 3 à 5, caractérisé en ce qu'on décompose l'agent de peroxyde éventuellement non converti avant qu'on distille.

7. Procédé selon l'une des revendications 1 à 2, caractérisé en ce que l'agent d'oxydation est un gaz contenant de l'oxygène moléculaire.

8. Procédé selon la revendication 7, caractérisé en ce que l'agent d'oxydation est de l'air.

9. Procédé selon l'une des revendications 7 à 8, caractérisé en ce que, pendant 0,5 à 10 heures, on fait passer 0,1 à 5 NL — calculés comme oxygène moléculaire — par heure et par kg de benzaldéhyde.

10. Procédé selon l'une des revendication 1 à 9, caractérisé en ce que le traitement avec un agent d'oxydation est effectué à une température située entre 20 et 200°C.